Europäisches Patentamt

European Patent Office

Office européen des brevets

(11) Publication number: 0 339 632
A2

# EUROPEAN PATENT APPLICATION

(21) Application number: 89107622.6

(22) Date of filing: 27.04.89

(51) Int. Cl.4: G01N 33/574 , G01N 33/543 , C12P 21/00

(30) Priority: 28.04.88 JP 105598/88

(43) Date of publication of application:
02.11.89 Bulletin 89/44

(84) Designated Contracting States:
DE FR GB

(71) Applicant: KYOWA HAKKO KOGYO
KABUSHIKI KAISHA
6-1, Ohte-machi 1-chome
Chiyoda-ku Tokyo 100(JP)

(72) Inventor: Yoshida, Hajime
9-18, Isobe
Sagamihara-shi Kanagawa(JP)
Inventor: Shitara, Kenya
3-9-11, Nakamachi
Machida-shi Tokyo(JP)

(74) Representative: Kinzebach, Werner, Dr. et al
Patentanwäite Reitstötter, Kinzebach und
Partner Sternwartstrasse 4 Postfach 86 06 49
D-8000 München 86(DE)

(54) Method of assaying human cancer antigens.

(57) A method of determining the human cancer antigen level in a serum sample by sandwich-technique enzyme immunoassay which comprises using, as the first antibody, a mixture of anti-human pulmonary adenocarcinoma monoclonal antibody ALC-186 and anti-human gastric cancer monoclonal antibody AMC-462 or a mixture of anti-human pulmonary adenocarcinoma monoclonal antibody ALC-186 and anti-human pulmonary squamous cell carcinoma monoclonal antibody SLC-454, immobilizing said first antibody on a solid phase, allowing a human serum sample to contact with said first antibody for causing a cancer antigen or antigens in the serum to be bound to said first antibody, then allowing a mixture of peroxidase-labeled anti-human pulmonary adenocarcinoma monoclonal antibody ALC-186 and β-galactosidase-labeled anti-human gastric cancer monoclonal antibody AMC-462, when the first antibody is a mixture of anti-human pulmonary adenocarcinoma monoclonal antibody ALC-186 and anti-human gastric cancer monoclonal antibody AMC-462, or a mixture of peroxidase-labeled anti-human pulmonary adenocarcinoma monoclonal antibody ALC-186 and β-galactosidase-labeled anti-human pulmonary squamous cell carcinoma monoclonal antibody SLC-454, when the first antibody is a mixture of anti-human pulmonary adenocarcinoma monoclonal antibody ALC-186 and anti-human pulmonary squamous cell carcinoma SLC-454, which is used each as the second antibody, to be bound to said cancer antigen or antigens, assaying the thus-bound β-galactosidase-labeled second antibody by addition of a substrate for β-galactosidase followed by enzyme activity measurement, and then assaying the bound peroxidase-labeled second antibody by addition of a substrate for peroxidase followed by enzyme activity measurement.

EP 0 339 632 A2

EP 0 339 632 A2

# METHOD OF ASSAYING HUMAN CANCER ANTIGENS

## FIELD OF THE INVENTION

This invention relates to a method useful in the field of clinical diagnosis and provides a method of assaying human cancer antigens in the serum which is applicable to clinical human cancer diagnosis.

## BACKGROUND OF THE INVENTION

The anti-human pulmonary adenocarcinoma monoclonal antibody ALC-186 (hereinafter briefly referred to as ALC-186) is a monoclonal antibody isolated by the present inventors. In a serodiagnostic system which uses this antibody, the percentage of positive reactions among pulmonary adenocarcinoma cases is 48% (EP-A-0218257).

The anti-human gastric cancer monoclonal antibody AMC-462 (hereinafter briefly referred to as AMC-462) is also a monoclonal antibody isolated by the present inventors. In a serodiagnostic system which uses this antibody, the percentage of positive reactions among gastric cancer cases is 18.6% (EP-A-0253646).

The anti-human pulmonary squamous cell carcinoma monoclonal antibody SLC-454 (hereinafter briefly referred to as SLC-454) is another monoclonal antibody isolated by the present inventors. In a serodiagnostic system which uses this antibody, the percentage of positive reactions among pulmonary squamous cell carcinoma cases is 27.5% (EP-A-0252769).

While a method of detecting a plurality of prostatic cancer antigens in one and the same system is already known (U.S. Patent 4,690,890), a method for the detection of other cancer antigens in one and the same system has not been described.

The serodiagnostic system for pulmonary adenocarcinoma which uses ALC-186, the serodiagnostic system for gastric cancer which uses AMC-462 and the serodiagnostic system for pulmonary squamous cell carcinoma which uses SLC-454, each referred to in the above, are already of great clinical importance. Nevertheless, in cancer diagnosis using sera, a diagnostic method giving a still higher percentage of positive reactions is desired.

The present inventors have already established a serodiagnostic system in which ALC-186 and the anti-human gastric adenocarcinoma monoclonal antibody AMC-382 are each used as the first antibody and peroxidase-labeled derivatives thereof are each used as the second antibody (EP-A-0242727). They have also established a serodiagnostic system in which ALC-186 and the anti-human pulmonary squamous cell carcinoma monoclonal antibody SLC-1 are each used as the first antibody and peroxidase-labeled derivatives thereof are each used as the second antibody, and have disclosed that these systems can assay adenocarcinoma antigens in sera efficiently with high positive reaction percentages (JP-A-62-238464; the term JP-A" as used herein means "an unexamined published Japanese patent application"). Although these systems give the percentage of positive reactions among cancer patients and are thus useful as initial tests, they are disadvantageous in that cancer species cannot be specifically identified in actual clinical testing using such systems since only one color developing system is used for each system, the system is non-species specific and accordingly a determination cannot be made as to which antibody is the cause of color development.

## SUMMARY OF THE INVENTION

The present inventors found that an assay method comprising using a mixture of ALC-186 and AMC-462 or a mixture of ALC-186 and SLC-454 as the first antibody and a mixture of ALC-186 and AMC-462 respectively labeled with two different enzymes, namely peroxidase-labeled ALC-186 and $\beta$-galactosidase-labeled AMC-462, or a mixture of ALC-186 and SLC-454 respectively labeled with these enzymes, namely peroxidase-labeled ALC-186 and $\beta$-galactosidase-labeled SLC-454, as the case may be, as the second antibody, to this mixture a substrate for $\beta$-galactosidase for color development is added, the color so developed is measured, the system is thoroughly washed, then a substrate for peroxidase for color development is added and the color so developed is measured. This provides for a markedly higher

2

positive rate with sera from cancer patients as compared with the conventional assay methods using the respective monoclonal antibodies as such and alone. It further enables one to discriminate between the antibodies relevant to the respective colors and thereby ascertain the cancer species involved.

Thus the invention provides a method of determining the human cancer antigen level in a serum sample by a sandwich-technique enzyme immunoassay which comprises using a mixture of ALC-186 and AMC-462 or a mixture of ALC-186 and SLC-454 as the first antibody, immobilizing said first antibody on a solid phase, allowing a human serum sample to contact the first antibody for causing a cancer antigen or antigens if present in the serum to be bound to the first antibody, then allowing a mixture of peroxidase-labeled ALC-186 and 8-galactosidase labeled AMC-462, when the first antibody is a mixture of ALC-186 and AMC-462, or a mixture of peroxidase-labeled ALC-186 and $\beta$-galactosidase-labeled SLC-454, when the first antibody is a mixture of ALC-186 and SLC-454, which is used as the second antibody, to be bound to the first antibody-bound cancer antigen or antigens, assaying the second antibody thus bound by first adding a substrate for $\beta$-galactosidase for color development, followed by color measurement and, after thorough washing, further adding a substrate for peroxidase for color development, followed by color measurement.

## DETAILED DESCRIPTION OF THE INVENTION

In accordance with the invention, a plurality of cancer antigens in a serum sample can be assayed in one and the same system. The monoclonal antibodies ALC-186, AMC-462 and SLC-454 are disclosed in EP-A-0218257 or U.S. Patent Serial No. 262,042, EP-A-0253646 or U.S. Patent Serial No. 070,071 and EP-A-0252769 or U.S. Patent Serial No. 070,052 (the inventors being the same as in the instant application) and can be produced using the hybridoma cell lines ALC-186, AMC-462 and SLC-454 which can produce the respective monoclonal antibodies. The hybridoma cell line ALC-186 has been deposited at the National Collection of Animal Cell Culture (Great Britain) under the deposit number NCACC 85082903 and the hybridoma cell lines AMC-462 and SLC-454 have been deposited at the European Collection of Animal Cell Culture (Great Britain) under the deposit numbers ECACC 86050801 and ECACC 86070306 under the Budapest Treaty, respectively.

The process for producing monoclonal antibodies ALC-186, AMC-462 and SLC-454 is as follows.

Eight- to ten-week-old female BALB/c or nude mice treated with pristane [intraperitoneally administered with 0.5 m$\ell$ of 2,6,10,14-tetramethylpentadecane (pristane) and fed for 1- to 2-weeks] are intraperitoneally injected with hybridomas of the cell line ALC-186, AMC-462 or SLC-454 at a dose of $2\text{-}4 \times 10^{5\,-7}$ cells per animal. In 10-21 days, the hybridomas cause ascites tumor in the mice.

In the case of monoclonal antibody ALC-186 or SLC-454, which belongs to the class IgM, the ascitic fluid is collected from such a mouse, subjected to salting out with 50% ammonium sulfate, dialyzed against phosphate-buffered saline (a solution containing, on the per-liter basis, 1.83 g of disodium phosphate, 0.21 g of monopotassium phosphate and 7.65 g of sodium chloride, pH 7.2; hereinafter referred to as PBS) supplemented with 0.5 M sodium chloride, and passed through a Cellulofine GCL 2000 (Seikagaku Kogyo) column (bed volume 750 m$\ell$) at a flow rate of 15 m$\ell$/hr. An IgM fraction is collected and used as a purified monoclonal antibody.

In the case of AMC-462, which is an antibody of the class $IgG_1$, the ascitic fluid is collected from such a mouse, centrifuged (3,000 rpm, 5 minutes) for removing solids, subjected to salting out with 50% ammonium sulfate, dialyzed against 0.04 M phosphate buffer (pH 8.0, containing 0.03 M NaCl) and passed through a DE52 (Whatman) column. An IgG fraction is collected and used as a purified monoclonal antibody.

The protein quantity is estimated by the Folin method, followed by calculation based on the absorbance at 280 nm [1.0 ($OD_{280}$) is approximately equal to 1 mg of immunoglobulin per milliliter].

The monoclonal antibodies thus obtained are evaluated for specificity characteristics based on the reactivities to normal and tumor tissues and membrane components thereof derived from a variety of human organs obtained from a plurality of subjects, reactivities to a variety of cultured human normal or tumor cell lines or cultured human fetal cell lines or membrane components derived therefrom, reactivities to the previously known carcinoembryonic antigen (CEA) and reactivities to normal sera and sera from various patients as determined by the enzyme immunoassay method, fluorescent antibody method, immunohistological evaluation method (PAP method), etc.

Enzyme-labeling of the monoclonal antibody ALC-186 is performed using peroxidase as the enzyme, and enzyme-labeling of AMC-462 and SLC-454 using $\beta$-galactosidase as the enzyme, for instance by the periodic acid-aided crosslinking method [J. Histochem. Cytochem., 22, 1084-1091 (1971)].

The periodic acid-aided crosslinking with peroxidase is carried out, for example, as follows:

Peroxidase is dissolved in 1 mℓ of 0.3 M sodium bicarbonate buffer (pH 8.1), 0.1 mℓ of a 1% solution of fluoro-2,4-dinitrobenzene in ethanol and, to the mixture, 1 mℓ of 0.06 M periodic acid is added, and the reaction is allowed to proceed at room temperature for 30 minutes. The reaction mixture is dialyzed against 0.01 M carbonate buffer (pH 9.5) to give a dialyzate containing peroxidase having dinitrophenyl (DNP)-modified amino groups and aldehyde group-introduced sugar moieties. The antibody (5 mg) is added to the dialyzate, the reaction is allowed to proceed at room temperature for 2 hours, the reaction mixture is dialyzed against 0.01 M phosphate buffer (pH 7.2), and the dialyzate is subjected to Sephadex G100 column chromatography for gel filtration. An active fraction recovered is used as the enzyme-labeled antibody.

Suited for use as the substrate for peroxidase is an ABTS substrate solution [prepared by dissolving 550 mg of 2,2'-azinobis(3-ethylbenzothiazoline-6-sulfonic acid) diammonium salt in 1 liter of 0.1 M citrate buffer (pH 4.2) and adding, just prior to use, hydrogen peroxide to a concentration of 1 μℓ/mℓ]. The color developed is measured in terms of optical density at 415 nm ($OD_{415\ nm}$).

Suited for use as the substrate for β-galactosidase is a solution prepared by dissolving 5 mg of o-nitrophenol-β-D-galactopyranoside in 1 mℓ of phosphate buffer containing 2 mM $MgCl_2$ and 0.1 mg/mℓ bovine serum albumin (BSA). The color developed is measured in terms of $OD_{415\ nm}$.

Serodiagnosis is carried out in the following manner:

A mixture of a 10-100 μg/mℓ ALC-186 solution and a 10-100 μg/mℓ AMC-462 solution or a mixture of a 10-100 μg/mℓ ALC-186 solution and a 10-100 μg/mℓ SLC-454 solution is distributed in 50- to 100-μℓ portions into the wells of a 96-well plate for EIA. These monoclonal antibodies are preferably mixed so as to give a final concentration equal to each other. The plate is allowed to stand at 4°C to room temperature for 2 to 40 hours.

After washing the plate with PBS, 200 μℓ of 1% BSA-PBS is added to each well, and the plate is allowed to stand at 4°C to room temperature for 2 to 40 hours. This plate is washed well with PBS and, then, 1- to 100-fold dilutions of a serum sample or samples are distributed into the wells (50-00 μℓ per well). After standing at 4°C to room temperature for 2 to 40 hours, the plate is washed well with PBS.

Then, a mixture of a 10-100 μg/mℓ peroxidase-labeled ALC-186 solution and a 10-100 μg/mℓ β-galactosidase-labeled AMC-462 solution, when a mixture of ALC-186 and AMC-462 is used as the first antibody, or a mixture of a 10-100 μg/mℓ peroxidase-labeled ALC-186 solution and a 10-100 μg/mℓ β-galactosidase-labeled SLC-454 solution, when a mixture of ALC-186 and SLC-454 is used as the first antibody, is distributed in 50- to 100-μℓ portions into the wells. These enzyme-labeled monoclonal antibodies are preferably mixed so as to give a final concentration equal to each other. The plate is allowed to stand at room temperature for 10-30 minutes and then washed well. The substrate for β-galactosidase is added for color development, the $OD_{415\ nm}$ value is measured for each well, and the quantity of a first antigen in each serum sample is calculated based on the color intensity data obtained. Then, the plate is washed thoroughly, the substrate for peroxidase is added for color development, the $OD_{415\ nm}$ value for each well is measured, and the quantity of a second antigen is calculated based on the color intensity data obtained. A normal antigen level range is defined by comparing the thus-obtained serum antigen level data for sera from healthy subjects with those for sera from cancer patients. When a level determined is above such normal range, the relevant case is determined as cancer-positive.

In a serodiagnostic system of the present invention in which ALC-186 and AMC-462 are used in combination, high positive rates are obtained among pulmonary adenocarcinoma and gastric cancer. In the case of the combination use of ALC-186 and SLC-454, high positive rates are obtained among pulmonary adenocarcinoma and pulmonary squamous cell carcinoma.

In accordance with the present invention, serodiagnoses of human cancers can be made efficiently in a simple and easy manner.

The following examples are further illustrative of the present invention, but are not construed to limit the scope of the present invention.

## EXAMPLE 1

An equivolume mixture of a 10 μg/mℓ ALC-186 solution and a 10 μg/mℓ AMC-462 solution was distributed into the wells of a 96-well plate for EIA (Flow Laboratories) (100 per well) as the first antibody. The plate was allowed to stand at 4°C for 15 hours and then washed with PBS. 1% BSA-PBS was added to the wells (200 μℓ per well), the plate was allowed to stand at 4°C for 15 hours and then washed well with

4

PBS. Ten-fold dilutions of sera from healthy subjects (20 samples), sera from pulmonary cancer patients (23 samples), sera from gastric cancer patients (20 samples), serum from a breast cancer patient (1 sample) and serum from a malignant lymphoma patient (1 sample) and serum from a malignant lymphoma patient (1 sample) were added to the wells (50 $\mu\ell$ per well). The plate was allowed to stand at 4°C for 15 hours and then washed well with PBS. An equivolume mixture of a peroxidase-labeled ALC-186 solution (10 $\mu$g/m$\ell$) and a $\beta$-galactosidase labeled AMC-462 solution (10 $\mu$g/m$\ell$) was added to the wells (100 $\mu\ell$ per well) as the second antibody. The plate was allowed to stand at 4°C for 15 hours and washed well with PBS.

The $\beta$-galactosidase substrate solution was added to the wells (50 $\mu\ell$ per well) and after 30 minutes of reaction at room temperature, the color developed in each well was determined by measuring the absorbance at 415 nm ($OD_{415\ nm}$) and the quantity of the cancer antigen recognized by AMC-462 was calculated.

Then, the plate was washed well, the peroxidase substrate solution was added to the wells (50 $\mu\ell$ per well) and, after 30 minutes of reaction, the color developed in each well was determined by measuring the absorbance at 415 nm ($OD_{415\ nm}$) and the quantity of the cancer antigen recognized by ALC-186 was calculated.

The results thus obtained are shown in Table 1.

The mean + 2 x SD (SD: standard deviation) for the 20 samples from healthy subjects as calculated for each assay system was taken as the cutoff value.

A system termed "combination assay system" was provided in which the sample was judged to be positive when it reacted in only one of the peroxidase and $\beta$-galactosidase systems. In such system, it is possible to make a combination assay of the cancer antigen recognizable by ALC-186 and the cancer antigen recognizable by AMC-462 on one plate. As shown in Table 1, remarkably high positive rates were obtained in the combination assay system among cancer cases in general, and in particular among pulmonary adenocarcinoma, gastric cancer and other cancer cases. Furthermore, it is also possible to determine or estimate the type of cancer based on whether the color development is found in the peroxidase system or in the $\beta$-galactosidase system, or in both.

Table 1

| Source of Serum Samples | Percent positiveness (positive samples/samples tested) | | |
|---|---|---|---|
| | Color-developing system | | |
| | Peroxidase | $\beta$-Galactosidase | Combination |
| Healthy subjects | 10.0 (2/20) | 10.0 (2/20) | 10.0 (2/20) |
| Cancer patients (total) | 20.6 (14/68) | 17.6 (12/68) | 38.2 (26/68) |
| Pulmonary cancer patients (total) | 26.1 (6/23) | 13.0 (3/23) | 34.8 (8/23) |
| Pulmonary adenocarcinoma patients | 62.5 (5/8) | 25.0 (2/8) | 87.5 (7/8) |
| Pulmonary squamous cell carcinoma patients | 0.0 (0/12) | 0.0 (0/12) | 0.0 (0/12) |
| Histological type unknown | 33.3 (1/3) | 33.3 (1/3) | 33.3 (1/3) |
| Gastric adenocarcinoma patients | 15.0 (3/20) | 30.0 (6/20) | 45.0 (9/20) |
| Breast cancer patients | 100.0 (1/1) | 0.0 (0/1) | 100.0 (1/1) |
| Malignant lymphoma patients | 0.0 (0/1) | 0.0 (0/1) | 0.0 (0/1) |

## EXAMPLE 2

An equivolume mixture of a 10 $\mu$g/m$\ell$ ALC-186 solution and a 10 $\mu$g/m$\ell$ SLC-454 solution was distributed into the wells of a 96-well plate for EIA (Flow Laboratories) (100 $\mu\ell$ per well) as the first antibody. The plate was allowed to stand at 4°C for 15 hours and then washed with PBS. 1% BSA-PBS was added to the wells (200 $\mu\ell$ per well), the plate was allowed to stand at 4°C for 15 hours and then washed well with PBS. Ten-fold dilutions of sera from healthy subjects (20 samples), sera from pulmonary cancer patients (23 samples), sera from gastric cancer patients (20 samples), serum from a breast cancer patient (1 sample) and serum from a malignant lymphoma patient (1 sample) were added to the wells (50 $\mu\ell$ per well). The plate was allowed to stand at 4°C for 15 hours and then washed well with PBS. An equivolume mixture of a peroxidase-labeled ALC-186 solution (10 $\mu$g/m$\ell$) and a $\beta$-galactosidase-labeled SLC-454 solution (10 $\mu$g/m$\ell$) was added to the wells (100 $\mu\ell$ per well) as the second antibody. The plate was allowed to stand at 4°C for 15 hours and washed well with PBS.

The $\beta$-galactosidase substrate solution was added to the wells (50 $\mu\ell$ per well) and after 30 minutes of reaction at room temperature, the color developed in each well was determined by measuring the absorbance at 415 nm ($OD_{415\ nm}$) and the quantity of the cancer antigen recognized by SLC-454 was calculated.

Then, the plate was washed well, the peroxidase substrate solution was added to the wells (50 $\mu\ell$ per well) and, after 30 minutes of reaction, the color developed in each well was determined by measuring the absorbance at 415 nm ($OD_{415\ nm}$) and the quantity of the cancer antigen recognized by ALC-186 was calculated.

The results thus obtained are shown in Table 2.

The mean + 2 x SD (SD: standard deviation) for the 20 samples from healthy subjects as calculated for each assay system was taken as the cutoff value.

In this system, it is possible to make a combination assay of the cancer antigen recognizable by ALC-186 and the cancer antigen recognizable by SLC-454 on one plate. As shown in Table 2, remarkably high positive rates were obtained in the combination assay system among cancer cases in general, in particular among pulmonary cancer, pulmonary adenocarcinoma and other cancer cases. Furthermore, it is also possible to determine or estimate the type of cancer based on whether the color development is found in the peroxidase system or in the $\beta$-galactosidase system, or in both.

Table 2

| Source of Serum Samples | Percent positiveness (positive samples/samples tested) | | |
|---|---|---|---|
| | Color-developing system | | |
| | Peroxidase | $\beta$-Galactosidase | Combination |
| Healthy subjects | 10.0 (2/20) | 0.0 (0/20) | 10.0 (2/20) |
| Cancer patients (total) | 23.5 (16/68) | 23.5 (16/68) | 44.1 (30/68) |
| Pulmonary cancer patients (total) | 26.1 (6/23) | 34.8 (8/23) | 56.5 (13/23) |
| Pulmonary adenocarcinoma patients | 62.5 (5/8) | 37.5 (3/8) | 87.5 (7/8) |
| Pulmonary squamous cell carcinoma patients | 0.0 (0/12) | 33.3 (4/12) | 33.3 (4/12) |
| Histological type unknown | 33.3 (1/3) | 33.3 (1/3) | 66.7 (2/3) |
| Gastric adenocarcinoma patients | 15.0 (3/20) | 0.0 (0/20) | 15.0 (3/20) |
| Breast cancer patients | 100.0 (1/1) | 0.0 (0/1) | 100.0 (1/1) |
| Malignant lymphoma patients | 0.0 (0/1) | 0.0 (0/1) | 0.0 (0/1) |

While the invention has been described in detail and with reference to specific embodiments thereof, it will be apparent to one skilled in the art that various changes and modifications can be made therein without departing from the spirit and scope thereof.

**Claims**

1. A method of determining the human cancer antigen level in a serum sample by a sandwich-technique enzyme immunoassay in a single immunoassay system which comprises the steps of:

(1) immobilizing a first antibody on a solid phase support in which the first antibody is a mixture of anti-human pulmonary adenocarcinoma monoclonal antibody ALC-186 and anti-human gastric cancer monoclonal antibody AMC-462, or

a mixture of anti-human pulmonary adenocarcinoma monoclonal antibody ALC-186 and anti-human pulmonary squamous cell carcinoma monoclonal antibody SLC-454;

6

(2) contacting the first antibody with a human serum sample thereby causing cancer antigen or antigens present in the serum and reactive with the first antibody to be bound to the first antibody;

(3) allowing a second antibody to be bound to the cancer antigen or antigens present in the serum sample in which the second antibody is

a mixture of peroxidase-labeled anti-human pulmonary adenocarcinoma monoclonal antibody ALC-186 and β-galactosidase-labeled anti-human gastric cancer monoclonal antibody AMC-462, when the first antibody is a mixture of anti-human pulmonary adeno-carcinoma monoclonal antibody ALC-186 and anti-human gastric cancer monoclonal antibody AMC-462, or

a mixture of peroxidase-labeled anti-human pulmonary adenocarcinoma monoclonal antibody ALC-186 and β-galactosidase-labeled anti-human pulmonary squamous cell carcinoma monoclonal antibody SLC-454, when the first antibody is a mixture of anti-human pulmonary adenocarcinoma monoclonal antibody ALC 186 and anti-human pulmonary squamous cell carcinoma monoclonal antibody SLC-454;

(4) assaying the thus-bound β-galactosidase-labeled second antibody by the addition of a substrate for β-galactosidase followed by measuring the enzyme activity; and then

(5) assaying the bound peroxidase-labeled second antibody by addition of a substrate for peroxidase followed by measuring the enzyme activity and thereby determining the human cancer antigen level in the serum sample.

2. The method of claim 1, in which monoclonal antibody ALC-186 is of the IgM isotype and is from hybridoma cell line deposit number NCACC 85082903 and progeny.

3. The method of claim 1, in which monoclonal antibody AMC-462 is of the IgG isotype and is from hydridoma cell line deposit number ECACC 86050801 and progeny.

4. The method of claim 1, in which the monoclonal antibody SLC-454 is of the IgM isotype and is from hybridoma cell line deposit number ECACC 86070306 and progeny.

5. The method of claim 1, in which the enzyme activity in step (4) is measured by determining the optical density at 415 nm.

6. The method of claim 1, in which the enzyme activity in step (5) is measured by determining the optical density at 415 nm.

7. The method of claim 1, including the additional step of:

(6) comparing the enzyme activity and the serum antigen level measured in steps (4) and (5) from the patient's serum sample with the serum antigen level for sera from healthy subjects.

8. A method of identifying the human cancer antigen for pulmonary adenocarcinoma, gastric cancer or pulmonary squamouns cell carcinoma and distinguishing between these three antigens in a human patient's serum sample by a sandwich-technique enzyme immunoassay in a single immunoassay system, which method comprises the steps of :

(1) immobilizing a first antibody on a solid phase support in which the first antibody is:

a mixture of a monoclonal antibody for human pulmonary adenocaqrcinoma and a monoclonal antibody human gastric cancer, or

a mixture of the monoclonal antibody for human pulmonoary adenocarcinoma and a monoclonal antibody for human pulmonary squamous cell carcinoma

wherein monoclonal antibody for human pulmonary adenocarcinoma is of the IgM isotype and is from hybridoma cell line deposit nymber NCACC 85082903 and progeny, the monoclonal antibody for human gastric cancer is of the IgG isotype and is from hydridoma cell line deposit number ECACC 86050801 and progeny, and the monoclonal antibody for human pulmonary squamous cell carcinoma is of the IgM isotype and is from hybridoma cell line deposit number ECACC 86070306;

(2) contacting the first antibody with the patient's serum sample thereby causing cancer antigen or antigens present in the serum and reactive with the first antibody to be bound to the first antibody;

(3) allowing a second antibody to be bound to the cancer antigen or antigens present in the serum sample in which the second antibody is

a mixture of peroxidase-labeled anti-human pulmonary adenocarcinoma monoclonal antibody and β-galactosidase-labeled anti-human gastric cancer monoclonal antibody, as defined above, when the first antibody is a mixture of a monoclonal antibody for human pulmonary adenocarcinoma and a monoclonal antibody for human gastric cancer, or

a mixture of peroxidase-labeled anti-human pulmonary adenocarcinoma monoclonal antibody and β-galactosidase-labeled anti-human pulmonary squamous cell carcinoma monoclonal antibody, as defined above, when the first antibody is a mixture of a monoclonal antibody for human pulmonary adenocarcinoma and a monoclonal antibody for human pulmonary squamous cell carcinoma monoclonal antibody;

7

(4) assaying the thus-bound β-galactosidase-labeled second antibody by the addition of a substrate for β-galactosidase followed by measuring the enzyme activity; and then

(5) assaying the bound peroxidase-labeled second antibody by addition of a substrate for peroxidase followed by measuring the enzyme activity and thereby determining the human cancer antigen level in the serum sample.

9. The method of claim 8, including the additional step of:

(6) comparing the enzyme activity and the serum antigen level measured in steps (4) and (5) from the patient's serum sample with the serum antigen level for sera from healthy subjects.